# EUROPÄISCHE PATENTSCHRIFT

(11) **EP 0 613 658 B1**
(45) Veröffentlichungstag und Bekanntmachung des Hinweises auf die Patenterteilung: **12.09.2001**
(21) Anmeldenummer: 93810167.2
(22) Anmeldetag: 05.03.1993
(51) Int. Cl.: A61B 17/16, A61F 2/46

(54) **Vorrichtung zum Bestimmen des Verlaufs von Bohrungen in Knochen**
Apparatus for determining the course of drill-holes in bone
Dispositif pour déterminer la course de trous de forage dans l'os

(43) Veröffentlichungstag der Anmeldung: 07.09.1994
(73) Patentinhaber: Sulzer Orthopädie AG, 6340 Baar (CH)
(72) Erfinder: Rothenbühler, Ulrich, CH-3007 Bern (CH)
(74) Vertreter: Sulzer Management AG

(56) Entgegenhaltungen:
- WO-A-85/02535
- US-A- 2 181 746
- US-A- 3 017 887
- US-A- 4 599 999
- US-A- 5 112 336

## Beschreibung

Die Erfindung betrifft eine Vorrichtung zum Bestimmen des Verlaufs von Bohrungen in Knochen gemäss dem Oberbegriff von Anspruch 1. Eine solche vorrichtung ist beispiels weise aus der US-A-2 181 746 bekannt.

Zur Verankerung von Implantaten im menschlichen Körper, beispielsweise bei künstlichen Hüftgelenkpfannen, sind Löcher im Knochen erforderlich, um das Implantat mit Schrauben im Knochen zu befestigen. Zum Bohren der Löcher gelangen, wie aus der WO-A-85/02535 bekannt, Bohrlehren zur Anwendung, die eine zentrale Führungsbohrung sowie einen Handgriff zur manuellen Richtungsvorgabe aufweisen. Diese Bohrlehren werden unmittelbar in eine üblicherweise kugelkalottenförmige Ausnehmung im Knochen eingebracht, sodass ein Spiralbohrer, durch die Führungsbohrung der Bohrlehre geführt, ein Loch mit entsprechender Bohrrichtung im Knochen erzeugt. Bei zweiteiligen Hüftgelenkspfannen, die aus einem Pfannenkörper sowie einem Verankerungskörper bestehen, wird üblicherweise erst der Verankerungskörper in die kugelkalottenförmige Ausnehmung geschlagen und daraufhin die Löcher in den Knochen gebohrt, indem die Bohrlehre in die halbkugelförmige Ausnehmung des Verankerungskörpers eingelegt wird. Derart lassen sich jedoch nur radial zur halbkugelförmigen Ausnehmung verlaufende Bohrlöcher realisieren. Dies wirkt sich unter verschiedenen Gesichtspunkten nachteilig aus. Gewisse Verankerungskörper sind derart ausgelegt, dass die Lage der Knochenschraube idealerweise nicht radial zur halbkugelförmigen Ausnehmung des Verankerungskörpers verläuft. Weiter ist die Operationsstelle üblicherweise schlecht zugänglich und unübersichtlich, sodass eine nur radial zur halbkugelförmigen Ausnehmung verlaufende Bohrrichtung eine weitere Einschränkung der Zugänglichkeit beziehungsweise der Bewegungsfreiheit darstellt.

Der Erfindung liegt die Aufgabe zugrunde eine Bohrlehre zu schaffen, die bei eingebrachtem Verankerungskörper nebst einer radial zu dessen halbkugelförmigen Innenfläche verlaufenden Bohrrichtung auch weitere Bohrrichtungen zulässt, derart, dass der maximale Winkel zwischen Bohrrichtung und radialer Richtung begrenzt ist.

Erfindungsgemäss wird diese Aufgabe gelöst gemäss den kennzeichnenden Merkmalen von Anspruch 1. Die Unteransprüche beziehen sich auf weitere, vorteilhafte Ausführungsformen der erfindungsgemässen Vorrichtung.

Die Vorteile der Erfindung sind insbesondere darin zu sehen, dass die gelenkig mit der Bohrbüchse verbundene Hülse auf ein Durchgangsloch im Verankerungskörper auflegbar ist, zum Beispiel formschlüssig. Somit nimmt die Hülse eine definierte Lage ein, bezüglich der die Bohrbüchse schwenkbar ist. Ein Begrenzungsanschlag kann den Schwenkwinkel zwischen Bohrbüchse und Hülse beschränken. Dies ergibt den Vorteil, dass der Neigungswinkel der Bohrung bezüglich dem, üblicherweise radial zur halbkugelförmigen Ausnehmung des Verankerungskörpers verlaufenden Durchgangsloch begrenzt ist. Bei einem zu grossen Winkel zwischen radialer Richtung und Bohrrichtung besteht die Gefahr, dass der Kopf der Knochenschraube, mit der der Verankerungskörper am Knochen befestigt wird, über die halbkugelförmige Innenfläche des Verankerungskörpers vorsteht, was ein spielfreies Aufliegen des Pfannenkörpers im Verankerungskörper verhindert. Dies kann durch die erfindungsgemässe Bohrlehre verhindert werden.

Zur Verankerung von Implantaten, wie künstlichen Hüftgelenkpfannen, sind Löcher 7a in das Knochenmaterial 4 zu bohren. Dabei dürfen die Bohrachsen 5b,5c maximal um einen Winkel α von einer normal zum Verankerungskörper 3 verlaufenden Achse 5a abweichen. Eine Vorrichtung 12 zum Bestimmen des Verlaufs von Bohrungen 7a im Knochen 4 besteht aus einer Bohrlehre 1 mit Bohrbüchse 1c und Handgriff 1a sowie einer gelenkig mit der Bohrbüchse 1c verbundenen Hülse 2. Die Hülse 2 weist eine Auflagefläche 2b sowie ev. einen Fortsatz 2h auf, der formschlüssig auf ein Durchgangsloch 3a im Verankerungskörper 3 auflegbar ist. Während dem Aufliegen der Hülse 2 auf dem Durchgangsloch 3a lässt sich die Bohrbüchse 1c in einem festgelegten Winkelbereich α bewegen, wobei ein Begrenzungsanschlag 2a der Hülse 2 sowie ein Begrenzungsanschlag 1g der Bohrbüchse 1c den Winkelbereich α begrenzen.

Im folgenden wird die Erfindung anhand von Ausführungsbeispielen beschrieben. Es zeigen:
- Fig. 1: eine auf einen Verankerungskörper aufgesetze Bohrlehre;
- Fig. 2: eine zerlegte Bohrlehre.

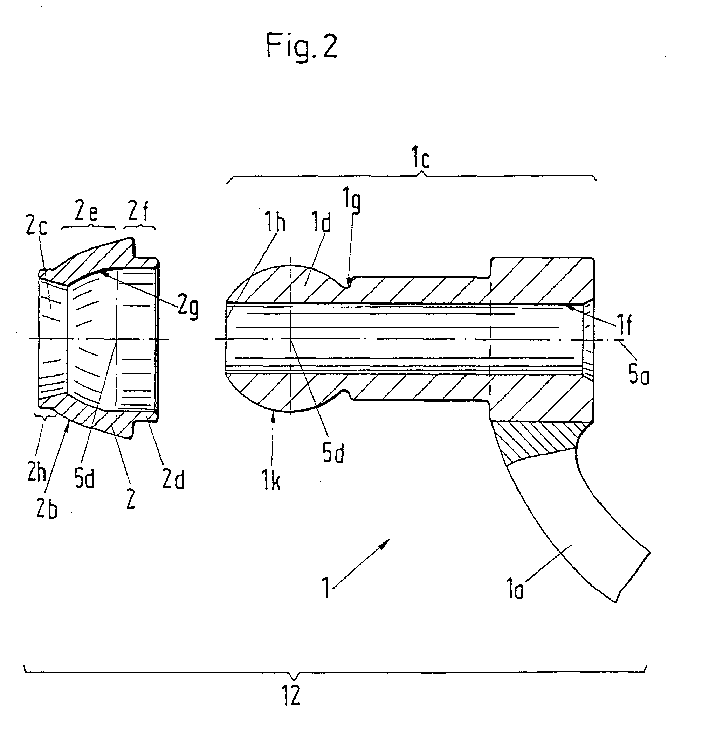

Fig. 2 zeigt eine Vorrichtung 12 zum Bestimmen des Verlaufs von Bohrungen, die im zerlegten Zustand aus zwei Teilen, einer Bohrlehre 1 sowie einer Hülse 2 besteht. Die Bohrlehre 1 besteht aus einer Bohrbüchse 1c, die in Richtung der Bohrachse 5a einen zylinderförmigen Führungskanal 1f für Bohrer 7b aufweist. An einem Ende der Bohrbüchse 1c ist ein Handgriff 1a befestigt, durch den die Lage der Bohrbüchse 1c und somit die Richtung der Bohrachse 5a manuell vorgebbar ist. Am entgegengesetzten Ende des Führungskanales 1f, im Bereich der Austrittsöffnung 1h, bildet die Bohrbüchse 1c einen mindestens teilweise konvex ausgebildeten Bereich 1d, mit einer kugelförmigen Oberfläche 1k. Die kugelförmige Oberfläche 1k wird von der Hülse 2 im zusammengesetzten Zustand teilweise umfasst, so dass sich eine kugelgelenkartige Verbindung zwischen der Bohrbüchse 1c und der Hülse 2 ergibt, mit einem gemeinsamen Drehzentrum 5d. Die Hülse 2 weist eine Auflagefläche 2b auf, die mindestens teilweise auf eine Öffnung im Verankerungskörper 3 zu liegen kommt, wobei die Auflagefläche 2b vorteilhafterweise derart ausgestaltet ist, dass diese formschlüssig auf die Öffnung im Verankerungskörpers 3 aufliegt. Die Auflagefläche 2b kann einen Fortsatz 2h aufweisen, dessen äussere Form derart auf die Öffnung des Verankerungskörpers 3 angepasst ist, dass die Hülse 2 in einer genau definierten Lage auf die Öffnung des Verankerungskörpers 3 zu liegen kommt. Die äussere Form des Fortsatzes 2h kann zum Beispiel zylinderförmig oder konisch sein. Die Hülse 2 weist eine Austrittsöffnung 2c auf. Die Hülse 2 weist einen mindestens teilweise konkaven, insbesondere kugelförmigen Innenraum auf zur Bildung des Kugelgelenkes mit der Bohrlehre 1. Im getrennten Zustand der Vorrichtung 12 weist die Hülse 2 anschliessend an den konkaven Innenraum 2e einen zylinderförmigen Innenraum 2f auf. Nach dem Zusammenführen von Hülse 2 und Bohrbüchse 1c wird der zylinderförmige Innenraum 2f um die kugelförmige Oberfläche 1k gepresst, sodass sich eine nicht lösbare Verbindung in Form eines Kugelgelenkes zwischen Hülse 2 und Bohrlehre 1 ergibt mit gemeinsamem Drehzentrum 5d. Dabei wirkt ein Begrenzungsanschlag 2d der Hülse 2 derart auf ein Begrenzungsanschlag 1g der Bohrbüchse 1c, dass die Auslenkung zwischen Hülse 2 und Bohrbüchse 1c begrenzt ist.

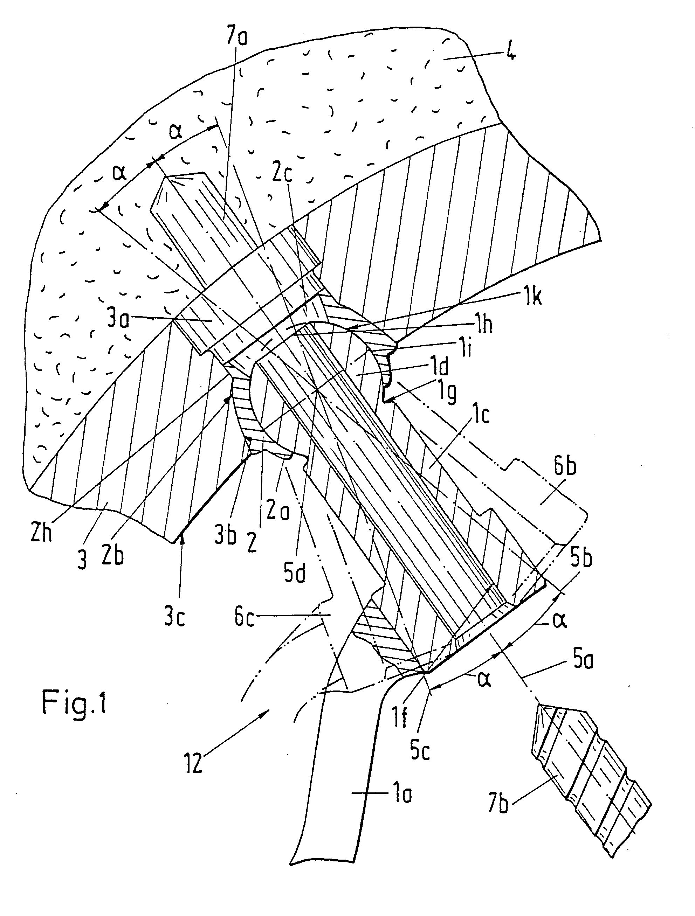

Fig. 1 zeigt die erfindungsgemässe Vorrichtung 12 zum Bestimmen des Verlauf von Bohrungen 7a im Zusammenhang mit der Implantation eines Verankerungskörpers 3 für Hüftgelenkpfannen. Nachdem eine kugelkalottenförmige Ausnehmung in das Knochenmaterial 4 getrieben ist, wird der Verankerungskörper 3 der Hüftgelenkpfanne in die Ausnehmung eingebracht. Danach werden Löcher 7a in den Knochen 4 gebohrt, die zur Aufnahme von Schrauben dienen, um das Implantat 3 im Knochen 4 zu verankern. Dargestellt ist ein Verankerungskörper 3 mit einem Durchgangsloch 3a, das sich gegen die dem Knochen abgewandte Seite hin verbreitert und eine Auflagefläche 3b bildet, auf die der Kopf der Schraube zu liegen komm. Zur Bestimmung der Bohrrichtung 5a des Bohrers 7b gelangt die Vorrichtung 12 zur Anwendung. Die Auflagefläche 2b der Hülse 2 ist derart ausgebildet, dass sie auf der Auflagefläche 3b des Verankerungskörpers 3 formschlüssig aufliegen kann. Die Hülse 2 wird über den Handgriff 1a zu einer Auflagefläche 3b geführt und derart in das Durchgangsloch 3a gedrückt, dass sich die Hülse 2 entsprechend der Auflagefläche 3b ausrichtet. Dabei kann ein Fortsatz 2h vorteilhaft sein, um die Lage der Hülse 2 bezüglich der Auflagefläche 3b präzise auszurichten und der Hülse einen zusätzlichen Halt zu verleihen. Bei einer derart festgelegten Lage der Hülse 2 lässt sich die Bohrbüchse 1c in einem durch die Begrenzungsanschläge 1g und 2a eingeschränkten Winkelbereich α bezüglich dem Drehzentrum 5d der Bohrachse schwenken. Die Bohrbüchse 1c ist in ihrer Mittellage mit Bohrachse 5a dargestellt, sowie in zwei Lagen maximaler Auslenkung, einer Stellung 6b der Bohrbüchse 1c mit Bohrachse 5b sowie einer Stellung 6c der Bohrbüchse 1c mit Bohrachse 5c. Somit lassen sich Bohrungen 7a in einem bezüglich der Auflagefläche 3b definierten Winkelbereich ausführen. Der eingeschränkte Winkelbereich der Bohrung 7a gewährleistet, dass ein auf der Auflagefläche 3b aufliegender Schraubenkopf nicht über die halbkugelförmige Innenfläche 3c des Verankerungskörpers 3 hinausragt. Ein nachträglich in den Verankerungskörper 3 eingefügter Pfannenkörper kann somit gleichmässig auf der Innenfläche 3c des Verankerungskörpers 3 aufliegen.

## Patentansprüche

1. Vorrichtung zum Vorgeben des Verlaufs von Bohrungen (7a) in Knochen (4), wobei die Bohrungen (7a) zur Verankerung von Implantaten wie künstlichen Hüftgelenkpfannen dienen, bestehend aus einer schwenkbaren Bohrlehre (1) mit einem Führungskanal (1f) zur Führung eines Bohrers (7b) sowie einem Handgriff (1a) zur manuellen Vorgabe der Richtung (5a) des Führungskanals (1f), wobei der Führungskanal (1f) durch eine Bohrbüchse (1c) gebildet wird, die im Bereich ihrer Austrittsöffnung (1h) mit einer Hülse (2) gelenkig verbunden ist, welche Hülse (2) eine Auflagefläche (2b) aufweist, um die Hülse (2) während dem Bohren auf einen Körper (3) aufzulegen, und wobei sowohl die Hülse (2) als auch die Bohrbüchse (1c) einen Begrenzungsanschlag (2a,1g) aufweisen, welche den Bereich des Schwenkwinkels (α) zwischen der Hülse (2) und der Bohrbüchse (1c) begrenzen, **dadurch gekennzeichnet, dass** die Hülse (2) eine konkav ausgebildete Innenfläche (2g) und die Bohrbüchse (1c) im Bereich ihrer Austrittsöffnung (1h) eine konvex ausgebildete Aussenfläche aufweisen, die jeweils kugelförmig ausgebildet sind und zusammen ein kugelförmiges Gelenk bilden, welches so ausgebildet ist, dass ein über den Handgriff (1a) auf den Körper (3) ausgeübter Druck über das kugelförmige Gelenk auf die Hülse (2) übertragbar ist und ein Aufdrücken der Auflagefläche (2b) der Hülse (2) auf den Körper (3) ermöglicht.

2. Vorrichtung nach Anspruch 1, **dadurch gekennzeichnet, dass** die Innenfläche (2g) der Hülse (2) die Aussenfläche (1k) der Bohrbüchse (1c) derart umschliesst, dass die Hülse (2) und die Bohrbüchse (1c) untrennbar miteinander verbunden sind.

3. Vorrichtung nach einem der Ansprüche 1 oder 2, **dadurch gekennzeichnet, dass** die Auflagefläche (2b) der Hülse (2) derart ausgestaltet ist, dass sie formschlüssig auf die Auflagefläche (3b) eines Durchgangsloches (3a) in dem Körper (3) auflegbar ist.

4. Vorrichtung nach einem der Ansprüche 1 bis 3, **dadurch gekennzeichnet, dass** die Auflagefläche (2b) im Bereich der Austrittsöffnung (2c) der Hülse (2) einen Fortsatz (2h) aufweist.

5. Vorrichtung nach einem der Ansprüche 1 bis 4, **dadurch gekennzeichnet, dass** die Aussenfläche des Fortsatzes (2h) zylinderförmig oder konisch ausgebildet ist.

6. Vorrichtung nach Anspruch einem der Ansprüche 1 bis 5, **dadurch gekennzeichnet, dass** der Begrenzungsanschlag (2a) der Hülse (2) sowie der Begrenzungsanschlag (1g) der Bohrbüchse (1c) rotationssymmetrisch bezüglich der Achse (5a) der Bohrbüchse (1c)ausgebildet sind, wenn sich die Bohrbüchse (1c) in der in Mittellage zwischen den Begrenzungsanschlägen (2a) befindet.

## Claims

1. A device for determining the path of drill holes (7a) in bone (4), wherein the drill holes (7a) are used to attach implants such as artificial acetabula, consisting of a swivelling hole gauge (1) having a guide channel (1f) for guiding a drill (7b) and also a handle (1a) for the manual determination of the direction (5a) of the guide channel (1f), wherein the guide channel (1f) is formed by a drill bush (1c) which in the region of its outlet aperture (1h) is connected in articulated manner with a socket (2), which socket (2) comprises a bearing face (2b) in order to lay the socket on a member (3) during drilling, and wherein both the socket (2) and the drill bush (1c) have a limit stop (2a, 1g), which limit the range of the swivel angle (α) between the socket (2) and the drill bush (1c),
**characterised in that** the socket (2) has a concave inner face (2g) and the drill bush (1c) has a convex outer face in the region of its outlet aperture (1h), which each have a spherical construction and together form a spherical joint, which is designed so that pressure exerted via the handle (1a) on the member (3) can be transmitted via the spherical joint to the socket (2) and enables the bearing face (2b) of the socket (2) to be pressed onto the member (3).

2. A device according to Claim 1,
**characterised in that** the inner face (2g) of the socket (2) surrounds the outer face (1k) of the drill bush (1c) in such a manner that the socket (2) and the drill bush (1c) are inseparably connected to one another.

3. A device according to one of Claims 1 or 2,
**characterised in that** the bearing face (2b) of the socket (2) is constructed in such a manner that it can be laid with form fit on the bearing face (3b) of a through-hole (3a) in the member (3).

4. A device according to one of Claims 1 to 3,
**characterised in that** the bearing face (2b) has a prolongation (2h) in the region of the outlet aperture (2c) of the socket (2).

5. A device according to one of Claims 1 to 4,
**characterised in that** the outer face of the prolongation (2h) has a cylindrical or conical design.

6. A device according to Claim one of Claims 1 to 5,
**characterised in that** the limit stop (2a) of the socket (2) and also the limit stop (1g) of the drill bush (1c) are constructed rotationally symmetrically with respect to the axis (5a) of the drill bush (1c) when the drill bush (1c) is situated in the central position between the limit stops (2a).

## Revendications

1. Dispositif pour consigner la course de forages (7a) dans des os (4), où les forages (7a) sont destinés à l'ancrage d'implants comme de coques acétabulaires artificiel-les, constitué d'un gabarit de perçage pivotant (1) avec un canal de guidage (1f) pour le guidage d'un foret (7b) ainsi que d'une poignée (1a) pour la consigne manuelle de la direction (5a) du canal de guidage (1f), où le canal de guidage (1f) est formé par un manchon de forage (1c) qui est relié d'une manière articulée au voisinage de son ouverture de sortie (1h) à une douille (2), ladite douille (2) présentant une face d'application (2b) pour appliquer la douille (2) pendant le forage sur un corps (3), et où à la fois la douille (2) et aussi le manchon de forage (1c) présentent une butée de délimitation (2a, lg) qui délimitent la zone de l'angle de pivotement (α) entre la douille (2) et le manchon de forage (1c), **caractérisé en ce que** la douille (2) présente une face intérieure (2g) réalisée d'une manière concave et le manchon de forage (1c), au voisinage de son ouverture de sortie (1h), une face extérieure réalisée d'une manière convexe, qui sont respectivement réalisées d'une manière sphérique et forment ensemble une articulation sphérique qui est réalisée de façon qu'une pression exercée par la poignée (1a) sur le corps (3) puisse être transmise par l'articulation sphérique sur la douille (2) et permette une application par pression de la face d'application (2b) de la douille (2) sur le corps (3).

2. Dispositif selon la revendication 1, **caractérisé en ce que** la face intérieure (2g) de la douille (2) entoure la face extérieure (1k) du manchon de forage (1c) de façon que la douille (2) et le manchon de forage (1c) soient reliés entre eux d'une manière non séparable.

3. Dispositif selon l'une des revendications 1 ou 2, **caractérisé en ce que** la face d'application (2b) de la douille (2) est réalisée de façon qu'elle puisse être placée par concordance des formes sur la face d'application (3b) d'un trou traversant (3a) dans le corps (3).

4. Dispositif selon l'une des revendications 1 à 3, **caractérisé en ce que** la face d'application (2b) présente au voisinage de l'ouverture de sortie (2c) de la douille (2) un prolongement (2h).

5. Dispositif selon l'une des revendications 1 à 4, **caractérisé en ce que** la face extérieure du prolongement (2h) est réalisée d'une manière cylindrique ou conique.

6. Dispositif selon l'une des revendications 1 à 5, **caractérisé en ce que** la butée de délimitation (2a) de la douille (2) ainsi que la butée de délimitation (1g) du manchon de perçage (1c) sont réalisées d'une manière symétrique en rotation par rapport à l'axe (5a) du manchon de forage (1c) lorsque le manchon de forage (1c) se trouve dans la position médiane entre les butées de délimitation (2a).
